# EUROPEAN PATENT APPLICATION

(11) **EP 4 757 340 A1**
(43) Date of publication of application: **10.06.2026**
(21) Application number: 25214921.6
(22) Date of filing: 11.11.2025
(51) Int. Cl.: H04Q 9/00

(54) **SENSING DEVICE**

(30) Priority: 04.12.2024 US 202418969031
(71) Applicant: Life Safety Distribution GmbH, 1180 Rolle (CH)
(72) Inventor: AHN, Jephil, Charlotte, 28202 (US); BAEK, Hyungwoo, Charlotte, 28202 (US); BYON, Jaeseob, Charlotte, 28202 (US); LEE, Byungyong, Charlotte, 28202 (US); LEE, Haeyong, Charlotte, 28202 (US)
(74) Representative: Haseltine Lake Kempner LLP

(57) **Abstract**

A sensing device is disclosed. The sensing device comprises a sensor retainer comprising a first coil, a first sensor, and a first light communication tube, and a sensor cartridge detachably coupled to the sensor retainer and comprising a second coil, a second sensor, a second light communication tube. Further, at least one controller is configured to measure a current flowing through the first coil based at least on transmitted pulse of a predefined frequency, determine if the measured current is above a predefined threshold value, if the measured current is above the predefined threshold value, apply a constant frequency of power to the sensor cartridge via the first coil and the second coil, and perform a wireless communication between the at least one controller and the sensor cartridge via the first sensor and the second sensor using the first light communication tube and the second light communication tube.

## Description

### TECHNOLOGICAL FIELD

Example embodiments of the present disclosure relate generally to a gas detector, and more particularly, to a sensing device and a method thereof.

### BACKGROUND

Gas detectors are devices used for monitoring hazardous gases within or outside a confined space, but installation and maintenance of the gas detectors require careful attention and detail to ensure safety and functionality. When installing or replacing sensor cartridges, users must be mindful of the power connection and proper orientation. For flame-proof cartridges, the power must be disconnected to prevent risks, while intrinsically safe sensor cartridges require precise alignment of electrical connections. Incorrect installation can lead to damaged connections, increasing the risk of gas leaks or explosions, especially if handled by unqualified personnel. Additionally, exposure to water or foreign substances during or after installation can lead to oxidation of the connections, and thus potentially causing malfunctions.

The inventors identified numerous deficiencies and problems in in existing technologies and processes, which are the subjects of embodiments described herein. Through applied effort, ingenuity, and innovation, many of these deficiencies and problems have been solved by developing solutions that are included in embodiments of the present disclosure, many examples of which are described in detail herein.

### BRIEF SUMMARY

The following presents a summary of some example embodiments to provide a basic understanding of some aspects of the present disclosure. This summary is not an extensive overview and is intended to neither identify key or critical elements nor delineate the scope of such elements. It will also be appreciated that the scope of the disclosure encompasses many potential embodiments in addition to those here summarized, some of which will be further described in the detailed description that is presented later.

In an example embodiment, a sensing device is disclosed. The sensing device comprises a sensor retainer comprising a first coil, a first sensor, and a first light communication tube. Further, the sensing device comprises a sensor cartridge detachably coupled to the sensor retainer and comprising a second coil, a second sensor, and a second light communication tube. Further, the sensing device comprises at least one controller electrically coupled to the sensor retainer and communicatively coupled to the sensor cartridge. Further, the at least one controller is configured to measure a current flowing through the first coil based at least on transmitted pulse of a predefined frequency, determine if the measured current is above a predefined threshold value, if the measured current is above the predefined threshold value, apply a constant frequency of power to the sensor cartridge via the first coil and the second coil, and perform a wireless communication between the at least one controller and the sensor cartridge via the first sensor and the second sensor using the first light communication tube and the second light communication tube.

In some embodiments, the first coil corresponds to a transmitter coil and the second coil corresponds to a receiver coil.

In some embodiments, the first sensor and second sensor correspond to infrared data association (IrDA) sensors. Further, the first sensor and the second sensor are configured to transmit and receive optical signals through the first light communication tube and the second light communication tube to establish the wireless communication between the at least one controller and the sensor cartridge.

In some embodiments, the at least one controller is configured to transmit the pulse of the predefined frequency to the first coil for a predefined period of time.

In some embodiments, the at least one controller is configured to apply the constant frequency of power to the sensor cartridge via the first coil and the second coil for powering the sensor cartridge.

In some embodiments, a distal end of the first light communication tube is aligned with a proximal end of the second light communication tube to provide a light communication path for transmitting and receiving the optical signal from the first sensor to the second sensor and/or from the second sensor to the first sensor.

In some embodiments, the first light communication tube and the second light communication tube each correspond to a tempered glass tube to provide a flame-resistant path for transmitting and receiving the optical signal.

In some embodiments, the first light communication tube is encased within a flame proof layer, wherein the flame proof layer is made of at least one of an epoxy or cement material. In some embodiments, the first light communication tube and the first flame proof layer are enclosed within a protective shell to prevent the first light communication tube and the first flame proof layer from being displaced. In some embodiments, the protective shell is at least partially enclosed within a transmitter enclosure via an adhesive to provide flame resistance to the protective shell.

In another example embodiment, a method is disclosed. The method comprising. measuring, via at least one controller electrically coupled to a sensor retainer and communicatively coupled to a sensor cartridge, a current flowing through a first coil of the sensor retainer based at least on transmitted pulse of a predefined frequency; determining, via the at least one controller, if the measured current is above a predefined threshold value; applying, via the at least one controller, a constant frequency of power to the sensor cartridge via the first coil and a second coil of the sensor cartridge, if the measured current is above the predefined threshold value; and performing, via the at least one controller, a wireless communication between the at least one controller and the sensor cartridge via a first sensor of the sensor retainer and a second sensor of the sensor cartridge using a first light communication tube of the sensor retainer and a second light communication tube of the sensor cartridge.

The above summary is provided merely for purposes of summarizing some exemplary embodiments to provide a basic understanding of some aspects of the disclosure. Accordingly, it will be appreciated that the above-described embodiments are merely examples and should not be construed to narrow the scope or spirit of the disclosure in any way. It will be appreciated that the scope of the disclosure encompasses many potential embodiments in addition to those here summarized, some of which are further explained within the following detailed description and its accompanying drawings.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 illustrate a sectional view of a sensing device in accordance with an example embodiment of the present disclosure;
FIG. 2 illustrates another sectional view of the sensing device in accordance with an example embodiment of the present disclosure;
FIG. 3 illustrates communication between a sensor retainer and a sensor cartridge of the sensing device in accordance with an example embodiment of the present disclosure;
FIG. 4 illustrates an expanded-sectional view of the sensor retainer in accordance with an example embodiment of the present disclosure; and
FIG. 5 illustrates a flowchart showing a method of the sensing device in accordance with an example embodiment of the present disclosure.

### DETAILED DESCRIPTION

Some embodiments will now be described more fully hereinafter with reference to the accompanying drawings, in which some, but not all, embodiments of the present disclosure are shown. Indeed, various embodiments may be embodied in many different forms and should not be construed as limited to the embodiments set forth herein; rather, these embodiments are provided so that this disclosure will satisfy applicable legal requirements.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in dashed line for visibility of the underlying components.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in various embodiments," "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments or it may be excluded.

The present disclosure provides various embodiments of a sensing device. Embodiments of the present disclosure may comprise a sensor retainer. The sensor retainer may comprise a first coil, a first sensor, and a first light communication tube. Embodiments of the present disclosure may comprise a sensor cartridge that may be detachably coupled to the sensor retainer and may comprise a second coil, a second sensor, and a second light communication tube. Embodiments of the present disclosure may comprise at least one controller that may be electrically coupled to the sensor retainer and communicatively coupled to the sensor cartridge. Further, the at least one controller may be configured to measure a current flowing through the first coil based at least on transmitted pulse of a predefined frequency, determine if the measured current is above a predefined threshold value, if the measured current is above the predefined threshold value, apply a constant frequency of power to the sensor cartridge via the first coil and the second coil, and perform a wireless communication between the at least one controller and the sensor cartridge via the first sensor and the second sensor using the first light communication tube and the second light communication tube.

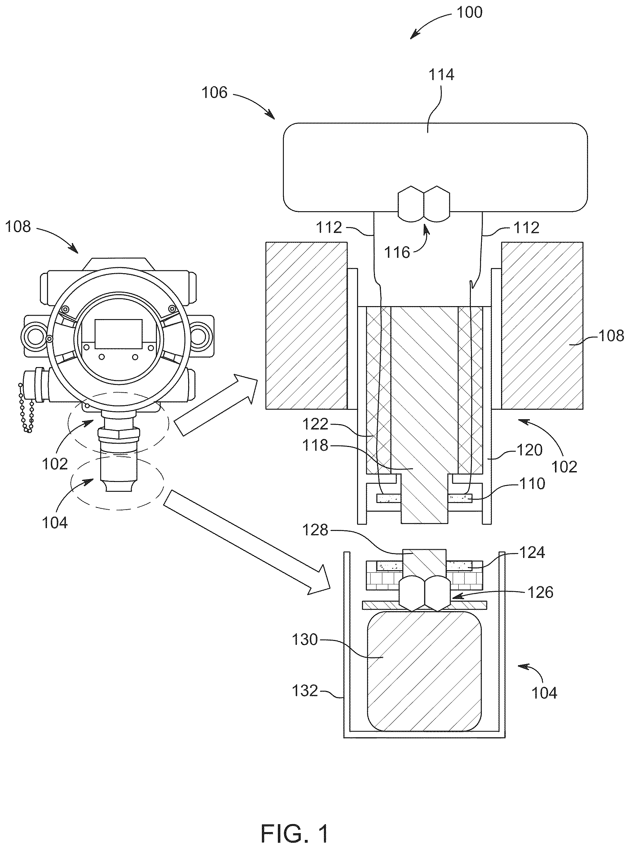

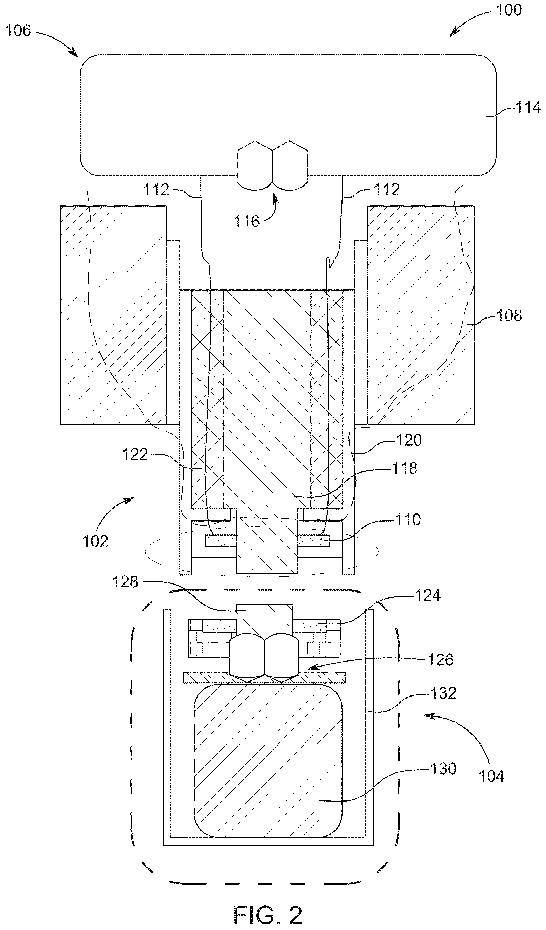

FIG. 1 illustrate a sectional view of a sensing device 100, in accordance with an example embodiment of the present disclosure. FIG. 2 illustrates another sectional view of the sensing device 100, in accordance with an example embodiment of the present disclosure.

In some embodiments, the sensing device 100 may comprise a sensor retainer 102, a sensor cartridge 104, and at least one controller 106. In some embodiments, the sensing device 100 may be installed within a facility (not shown). In some embodiments, the facility may correspond to industrial environments such as gas refineries, oil refineries, chemical processing plants, or other hazardous areas. In some embodiments, the sensing device 100 may be configured to determine presence of various gases within the facility. In some embodiments, the gases may include, but are not limited to, methane, propane, hydrogen sulfide, carbo monoxide, etc. In various examples, the sensing device 100 may be configured to determine concentration level of the gas present inside the facility.

In some embodiments, the sensing device 100 may comprise a transmitter enclosure 108. In some embodiments, the transmitter enclosure 108 of the sensing device 100 may be configured to encase one or more internal components associated with the sensing device 100. Further, the one or more internal components may comprise at least one of a power supply unit (not shown), processor/controller, communication cables, etc. In some embodiments, the transmitter enclosure 108 may be configured to safeguard the one or more internal components of the sensing device 100 from various environmental hazards. The environmental hazards may include, but are not limited to, dust, moisture, mechanical shock, etc. In some embodiments, the transmitter enclosure 108 may be constructed with various shapes. The shape may include, but are not limited to, cuboidal shape, cubical shape, spherical shape, cylindrical shape, etc. In some embodiments, the shape of the transmitter enclosure 108 may be selected in accordance with space within the facility, aesthetic considerations, or other functional requirements of the sensing device 100. In some embodiments, the transmitter enclosure 108 may be constructed with various materials. The material may include, but are not limited to, steel, aluminum, polycarbonate, reinforced fiber, etc. The material for making the transmitter enclosure 108 may be selected to provide strength and durability to the sensing device 100 in various environments.

In some embodiments, the sensing device 100 may comprise the sensor retainer 102. In some embodiments, the transmitter enclosure 108 may comprise the sensor retainer 102. In some embodiments, the sensor retainer 102 of the sensing device 100 may be configured to enable coupling of the sensor cartridge 104 with the transmitter enclosure 108 of the sensing device 100. In some embodiments, the sensor retainer 102 may be constructed with the transmitter enclosure 108 through various processes. The process may include, but is not limited to, drilling, cutting, molding, casting, etc. In one example, the transmitter enclosure 108 may comprise at least one sensor retainer 102. In another example, the transmitter enclosure 108 may comprise a plurality of sensor retainers 102. In some embodiments, the sensor retainer 102 may be constructed on at least one side of the transmitter enclosure 108. Further, the at least one side of the transmitter enclosure 108 may be aligned with a target area within the facility.

In some embodiments, the sensor retainer 102 may be constructed with various shapes. The shapes may include, but are not limited to a cylindrical shape, cubical shape, cuboidal shape, hexagonal shape, or hemispherical shape. In some embodiments, the shape of the sensor retainer 102 may be selected such that the sensor retainer 102 may have a minimalistic design and adaptable with various applications. In some embodiments, the sensor retainer 102 may be constructed with various materials. The materials may include, but are not limited to aluminum, steel, reinforced fiber, or alike. In some embodiments, the material for constructing the sensor retainer 102 may be selected such that the sensor retainer 102 may withstand various hazardous and extreme environmental conditions (e.g., high temperature, high mechanical stress, high humidity, etc.).

In some embodiments, the sensor retainer 102 may further comprise a first coil 110. In some embodiments, the first coil 110 of the sensor retainer 102 may correspond to a transmitter coil (i.e., an inductive element). In some embodiments, the first coil 110 may be configured to generate an electromagnetic field in proximity to a field of view (FOV) of the first coil 110. In one example, the FOV of the first coil 110 may correspond to a spatial region where the electromagnetic field is detectable and may interact with external objects or other components. In some embodiments, the sensing device 100 may comprise the power supply unit. In various examples, the power supply unit may comprise at least one of a battery or a direct power source. In some embodiments, the first coil 110 may be electrically coupled with the power supply unit of the sensing device 100 through at least two wires 112. In some embodiments, the power supply unit may be configured to provide an electrical supply (initially with a lower frequency of power) to the first coil 110.

Furthermore, upon receiving the electrical supply from the power supply unit, the first coil 110 may be configured to generate the electromagnetic field due a principle of electromagnetic induction. The electromagnetic induction involves the flow of alternating current (AC) through the first coil 110, which produces a time-varying magnetic field (i.e., the generated electromagnetic field). In some embodiments, the sensor retainer 102 may comprise a first circuit board 114. In some embodiments, the first circuit board 114 may enable fabrication of one or more electronic/electrical components (e.g., the power supply unit, processors/controllers, and sensors) associated with the sensor retainer 102. In some embodiments, the first circuit board 114 may correspond to a printed circuit board (PCB). In some embodiments, the first circuit board 114 may be configured provide electrical connection between the one or more electronic/electrical components associated with the sensor retainer 102.

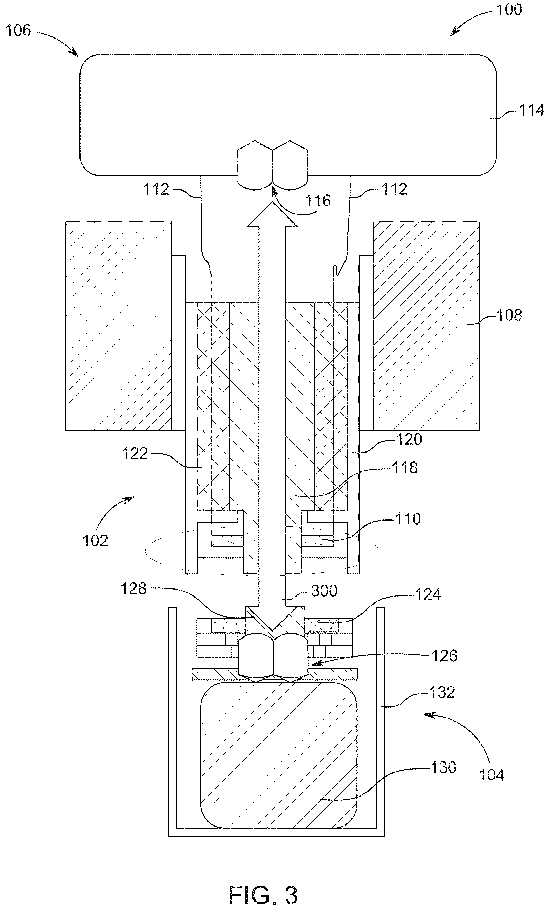

In some embodiments, the sensor retainer 102 of the sensing device 100 may comprise a first sensor 116. In some embodiments, the first sensor 116 may correspond to an optical sensor. In some embodiments, the first sensor 116 may be fabricated on the first circuit board 114. In some embodiments, the first sensor 116 of the sensor retainer 102 may be configured to transmit and receive optical signals 300 (FIG. 3) in its FOV. The FOV of the first sensor 116 may comprise a spatial area where the first sensor 116 may effectively detect and interact with the optical signals 300 provided by other components or devices. In some embodiments, the first coil 110 of the sensor retainer 102 may correspond to an infrared data associated (IrDA) sensor. The IrDA sensors are designed for short-range data communication using infrared light, typically in a wavelength range of 850 to 900 nanometers. In some embodiments, the first sensor 116 is configured to enable a wireless communication between the sensor retainer 102 and the sensor cartridge 104.

In some embodiments, the first sensor 116 may comprise at least one emitter and at least one detector. Further, the at least one emitter may be configured to generate and transmit the optical signals 300 in the FOV. Further, the at least one detector may be configured to receive the incoming optical signals 300. In some embodiments, the at least one emitter may correspond to a light-emitting diode (LED), laser diode, etc. In some embodiments, the at least one detector may correspond to a photodiode or phototransistor.

In some embodiments, the sensor retainer 102 may comprise a first light communication tube 118. In some embodiments, the first light communication tube 118 may be configured to be aligned with the transmitter enclosure 108. In some embodiments, the first light communication tube 118 may facilitate the wireless communication between the sensor retainer 102 and the sensor cartridge 104. In some embodiments, the first light communication tube 118 may be configured to receive the optical signals 300 generated by the first sensor 116. In some embodiments, the first light communication tube 118 may be configured to allow the optical signal to travel towards the sensor cartridge 104.

In some embodiments, the first light communication tube 118 may be constructed with various shapes. The shapes may include, but are not limited to, cylindrical shape, cuboidal shape, cubical shape, etc. In some embodiments, the first light communication tube 118 may be constructed with various materials. The material may include, but are not limited to, glass, fibers, polycarbonate, etc. The material for constructing the first light communication tube 118 may be selected such that the optical signals 300 may travel through the first light communication window without experiencing any losses (or experiencing minimal losses). In various examples, the material for making the first light communication tube 118 may be selected such that the first light communication tube 118 may withstand various extreme conditions such as fire, high mechanical stress, etc. In some embodiments, the first light communication tube 118 may comprise a proximal end and a distal end.

In some embodiments, the first light communication tube 118 may be encased within a protective shell 120. In some embodiments, the protective shell 120 may be at least partially enclosed within the transmitter enclosure 108 via an adhesive to provide flame resistance to the first light communication tube 118. In some embodiments, the protective shell 120 may be configured to safeguard the first light communication tube 118 from various environmental conditions. The conditions may include, but are not limited to, high temperature, high mechanical stress, humidity, etc. In some embodiments, the protective shell 120 may define an orifice. In some embodiments, the orifice of the protective shell 120 may be configured to accommodate the first light communication tube 118. In some embodiments, the orifice of the protective shell 120 may define one or more dimensions that may ensure that the protective shell 120 may properly accommodates the first light communication tube 118.

In some embodiments, the protective shell 120 may comprise a flame proof layer 122. In some embodiments, the flame proof layer 122 may be made of at least one of an epoxy, cement material, or other suitable compounds. In some embodiments, the flame proof layer 122 may be configured to encase the first light communication tube 118. The flame proof layer 122 filled within the space between the protective shell 120 and the first light communication tube 118 may be configured to secure the first light communication tube 118 within the protective shell 120. In various examples, the flame proof layer 122 filled within the space between the protective shell 120 and the first light communication tube 118 may be configured to provide a cushion to absorb shocks or vibrations. In various other examples, the material filled within the space between the protective shell 120 and the transparent window may act as a sealing agent, preventing moisture or other contaminants from entering the space.

As illustrated in FIG. 2, the sensor cartridge 104 may be configured to be detachably coupled to the sensor retainer 102 of the sensing device 100. In some embodiments, the detachable coupling of the sensor cartridge 104 with the sensor retainer 102 may facilitate replacement and maintenance of the sensor cartridge 104. In various examples, the sensor cartridge 104 may comprise one or more sensors (not shown). Further, the one or more sensors of the sensor cartridge 104 may be configured to detect presence of the gases and concentration of the gases within the facility. In some embodiments, the one or more sensors may correspond to at least one of infrared (IR) sensors, chemical sensors, electrochemical sensors, or other specialized detectors.

In some embodiments, the sensor cartridge 104 may be configured to be snapped together with the sensor retainer 102. In some embodiments, the sensor cartridge 104 may be configured to accommodate a predefined portion of the sensor retained within the sensor cartridge 104 upon coupling the sensor cartridge 104 with the sensor retainer 102. In some embodiments, the sensor cartridge 104 may comprise a second coil 124, a second sensor 126, and a second light communication tube 128. Further, the second coil 124 may correspond to a receiver coil. In some embodiments, upon coupling of the sensor cartridge 104 with the sensor retainer 102, the second coil 124 may be in close proximity to the first coil 110 and therefore may be configured to receive the electric power supply (i.e., having a lower frequency of power) through the electromagnetic field generated by the first coil 110.

In some embodiments, the sensor cartridge 104 may comprise a second circuit board 130. In some embodiments, the second circuit board 130 may be coupled with the receiver coil. Further, the second coil 124 may be configured to supply power to the second circuit board 130. Further, the second circuit board 130 may be configured to supply the electric power supply to various components (i.e., the second sensor 126) associated with the sensor cartridge 104. In some embodiments, the second sensor 126 may be fabricated with the second circuit board 130. In some embodiments, the second sensor 126 may correspond to the infrared data associated (IrDA) sensor. In some embodiments, the second sensor 126 may be configured to transmit/receive the optical signals 300.

In some embodiments, the sensor cartridge 104 may comprise the second light communication tube 128. Further, the sensor cartridge 104 may comprise another protective shell 132. In some embodiments, the another protective shell 132 may be configured to protect one or more components associated with the sensor cartridge 104. In some embodiments, the second light communication tube 128 of the sensor cartridge 104 may be encased within the another protective shell 132. Further, the second light communication tube 128 may be configured to enable the optical signals 300 to travel from the second sensor 126 and towards the first light communication tube 118. In some embodiments, the second light communication tube 128 may comprise a proximal end and a distal end. In some embodiments, the distal end of the first light communication tube 118 may be aligned with the proximal end of the second light communication tube 128 to provide a light communication path for transmitting and receiving the optical signal from the first sensor 116 to the second sensor 126 and/or from the second sensor 126 to the first sensor 116. In some embodiments, the first light communication tube 118 and the second light communication tube 128 each may correspond to a tempered glass tube to provide a flame-resistant path for transmitting and receiving the optical signal.

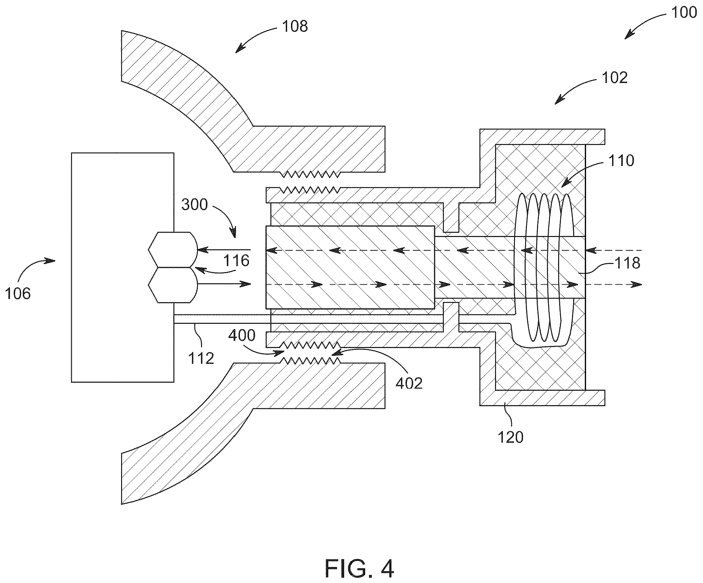

In some embodiments, the first sensor 116 and the second sensor 126 may be configured to enable wireless communication between the sensor retainer 102 and the sensor cartridge 104 through the first light communication tube 118 and the second light communication tube 128. In some embodiments, the sensing device 100 may comprise the at least one controller 106. In some embodiments, the at least one controller 106 may be fabricated over the first circuit board 114 of the sensor retainer 102. In some embodiments, the at least one controller 106 may be configured to control the wireless communication between the sensor retainer 102 and the sensor cartridge 104. It may be noted that a detailed explanation of the at least one controller 106 is described in FIG. 3 and FIG. 4.

FIG. 3 illustrates communication between the sensor retainer 102 and the sensor cartridge 104 of the sensing device 100, in accordance with an example embodiment of the present disclosure. FIG. 4 illustrates an expanded-sectional view of the sensor retainer 102, in accordance with an example embodiment of the present disclosure.

In some embodiments, the sensing device 100 may be configured to determine concentration level of the gas present inside the facility. In some embodiments, the sensing device 100 may comprise the sensor retainer 102. In some embodiments, the sensor retainer 102 of the sensing device 100 may be configured to enable coupling of the sensor cartridge 104 with the transmitter enclosure 108 of the sensing device 100. In various examples, the sensor retainer 102 may be detachably coupled with the transmitter enclosure 108 through a plurality of threads, as illustrated in FIG. 4.

In some embodiments, the sensor retainer 102 may comprise a plurality of external threads 400 (FIG. 4). In some embodiments, the plurality of external threads 400 of the sensor retainer 102 may be configured to enable coupling of the sensor retainer 102 with the transmitter enclosure 108. In some embodiments, the transmitter enclosure 108 may comprise a plurality of internal threads 402 (FIG. 4). In some embodiments, the sensor retainer 102 may be screwed in by a person with the transmitter enclosure 108, thereby securing the sensor retainer 102 with the transmitter enclosure 108 through the plurality of internal threads 402 and the plurality of external threads 400.

In some embodiments, the sensor retainer 102 may comprise the first coil 110. Further, the first coil 110 may be configured generate the electromagnetic field in proximity to an FOV of the first coil 110. Further, the first coil 110 may be electrically coupled to the power supply unit. Furthermore, upon receiving the electrical supply from the power supply unit, the first coil 110 may be configured to generate the electromagnetic field. In some embodiments, the sensor retainer 102 of the sensing device 100 may comprise the first sensor 116. In some embodiments, the first sensor 116 of the sensor retainer 102 may be configured to transmit and receive the optical signals 300 in its field of view (FOV). In some embodiments, the sensor retainer 102 may comprise the first light communication tube 118. In some embodiments, the first light communication tube 118 may be configured to receive the optical signals 300 generated by the first sensor 116. In some embodiments, the first light communication tube 118 may be configured to allow the optical signal to travel towards the sensor cartridge 104. In some embodiments, the sensor cartridge 104 may be configured to be detachably coupled to the sensor retainer 102 of the sensing device 100. In some embodiments, the sensor cartridge 104 may comprise the second coil 124, the second sensor 126, and the second light communication tube 128. In some embodiments, upon coupling of the sensor cartridge 104 with the sensor retainer 102, the second coil 124 may be configured to receive the electric power supply through the electromagnetic field generated by the first coil 110. In some embodiments, the first sensor 116 and the second sensor 126 may be configured to enable wireless communication between the sensor retainer 102 and the sensor cartridge 104 through the first light communication tube 118 and the second light communication tube 128.

In some embodiments, the at least one controller 106 may be electrically coupled to the sensor retainer 102 and communicatively coupled to the sensor cartridge 104. The at least one controller 106 may include suitable logic, input/ output circuitry, and communication circuitry that are operable to execute one or more instructions stored in a memory to perform predetermined operations. In one embodiment, the at least one controller 106 may be configured to decode and execute any instructions received from one or more other electronic devices or server(s). The at least one controller 106 may be configured to execute one or more computer-readable program instructions, such as program instructions to carry out any of the functions described in this description. Further, the at least one controller 106 may be implemented using one or more technologies known in the art. Examples of the at least one controller 106 include, but are not limited to, one or more general purpose controllers and/or one or more special purpose controllers.

In some embodiments, the at least one controller 106 may be configured to measure a current flowing through the first coil 110 based at least on transmitted pulse of a predefined frequency. Further, the at least one controller 106 is configured to transmit the pulse of the predefined frequency to the first coil 110 for a predefined period of time. Further, the predefined period of time may correspond to 0.5-1 second. Further, the at least one controller 106 may be configured to determine if the measured current is above a predefined threshold value. In some embodiments, if the measured current is above the predefined threshold value, indicating that the sensor cartridge 104 is properly attached and ready for operation, then the at least one controller 106 may be configured to apply a constant frequency of power to the sensor cartridge 104 via the first coil 110 and the second coil 124. Further, the at least one controller 106 may be configured to apply the constant frequency of power to the sensor cartridge 104 via the first coil 110 and the second coil 124 for powering the sensor cartridge 104. In some embodiments, the at least one controller 106 may be configured to perform a wireless communication between the at least one controller 106 and the sensor cartridge 104 via the first sensor 116 and the second sensor 126 using the first light communication tube 118 and the second light communication tube 128. In some embodiments, the first sensor 116 and the second sensor 126 may be configured to transmit and receive the optical signals 300 through the first light communication tube 118 and the second light communication tube 128 to establish the wireless communication between the at least one controller 106 and the sensor cartridge 104.

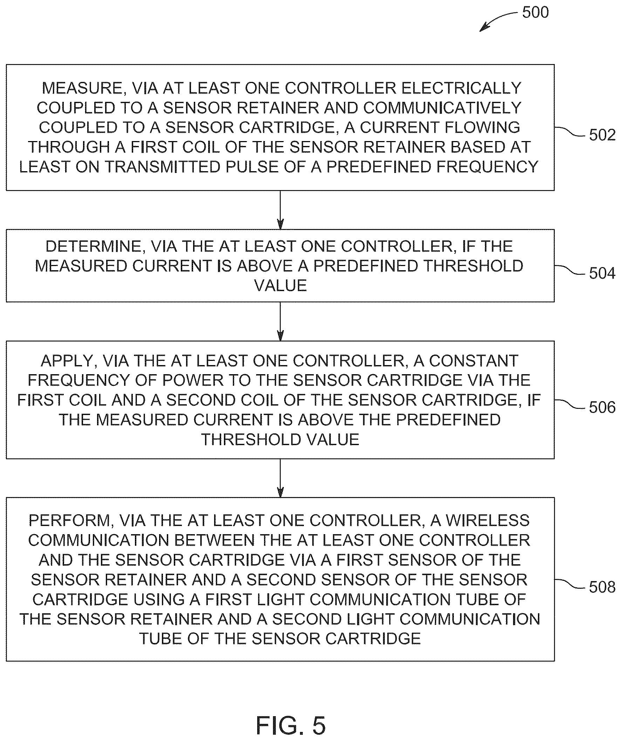

FIG. 5 illustrates a flowchart showing a method 500 of the sensing device 100, in accordance with an example embodiment of the present disclosure.

At operation 502, the at least one controller 106 electrically coupled to the sensor retainer 102 and communicatively coupled to the sensor cartridge 104 may be configured to measure a current flowing through the first coil 110 of the sensor retainer 102 based at least on transmitted pulse of a predefined frequency. Further, the first coil 110 may correspond to the transmitter coil. Further, the first coil 110 may be configured generate the electromagnetic field in proximity to an FOV of the first coil 110. Further, the predefined period of time may correspond to 0.5-1 second. In some embodiments, the sensor cartridge 104 may be configured to be detachably coupled to the sensor retainer 102 of the sensing device 100.

For example, a sensing device 100 may be utilized in a gas detector device. The sensing device 100 may comprise a sensor retainer 102. The sensor retainer 102 of the gas detector device includes the first coil 110, which transmits a pulse of a predefined frequency to enable measurement of the current flowing through the first coil 110. The sensing device 100 comprises a sensor cartridge 104 having one or more sensors configured to be utilized during operations of the gas detector device.

At operation 504, the at least one controller 106 may be configured to determine if the measured current is above a predefined threshold value. In some embodiments, the current may be supplied by the power supply unit to the first coil 110. Further, the first coil 110 may be electrically coupled to the power supply unit. Furthermore, upon receiving the electrical supply from the power supply unit, the first coil 110 may be configured to generate the electromagnetic field.

For example, the at least one controller 106 measures the current in the first coil 110 to determine if the current exceeds the threshold value. If the determined current exceeds the threshold value, the at least one controller 106 determines that the sensor cartridge 104 is properly attached and ready for operation.

At operation 506, if the measured current is above the predefined threshold value, then the at least one controller 106 may be configured to apply a constant frequency of power to the sensor cartridge 104 via the first coil 110 and the second coil 124. Further, the at least one controller 106 may be configured to apply the constant frequency of power to the sensor cartridge 104 via the first coil 110 and the second coil 124 for powering the sensor cartridge 104.

For example, in the gas detector device, if the at least one controller 106 determines the current is above the predefined threshold value, the at least one controller 106 applies a constant frequency of power to keep the sensors in the cartridge active.

At operation 508, the at least one controller 106 may be configured to perform a wireless communication between the at least one controller 106 and the sensor cartridge 104 via the first sensor 116 and the second sensor 126 using the first light communication tube 118 and the second light communication tube 128. In some embodiments, the first sensor 116 and the second sensor 126 may be configured to transmit and receive the optical signals 300 through the first light communication tube 118 and the second light communication tube 128 to establish the wireless communication between the at least one controller 106 and the sensor cartridge 104. In various embodiments, the optical signals 300 enable transmission of information between the sensor cartridge 104 and the at least one controller 106. For example, the optical signals 300 may enable transmission of a detected gas level from the sensor cartridge 104 to the at least one controller 106.

For example, in the gas detector device, the first sensor 116 in the sensor retainer 102 and the second sensor 126 in the sensor cartridge 104 comprises a first light communication tube 118 and a second light communication tube 128 to wirelessly transmit and receive data.

Embodiments of the present disclosure offer significant advantages in enhancing the efficiency and reliability of sensor-based systems. The integration of the first coil 110, first sensor 116, and first light communication tube 118 within the sensor retainer 102, in conjunction with the second coil 124, second sensor 126, and second light communication tube 128 within the sensor cartridge 104, ensures robust and flexible operation of the sensing device 100. The at least one controller 106, that is electrically and communicatively coupled to both the sensor retainer 102 and the sensor cartridge 104, is configured to measure current flow through the first coil 110 based on a transmitted pulse of a predefined frequency allowing for precise determination of whether the measured current exceeds a predefined threshold value. Further, the at least one controller 106 applies a constant frequency of power to the sensor cartridge 104 via the first coil 110 and second coil 124, thereby maintaining optimal operational conditions of the sensing device 100.

Many modifications and other embodiments of the disclosure set forth herein will come to mind to one skilled in the art to which the present disclosure pertains having the benefit of the teachings presented in the foregoing descriptions and the associated drawings. Therefore, it is to be understood that the present disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A sensing device comprising:
a sensor retainer comprising:
a first coil;
a first sensor; and
a first light communication tube;
a sensor cartridge detachably coupled to the sensor retainer and comprising:
a second coil;
a second sensor; and
a second light communication tube; and
at least one controller electrically coupled to the sensor retainer and communicatively coupled to the sensor cartridge, wherein the at least one controller is configured to:
measure a current flowing through the first coil based at least on transmitted pulse of a predefined frequency;
determine if the measured current is above a predefined threshold value;
if the measured current is above the predefined threshold value, apply a constant frequency of power to the sensor cartridge via the first coil and the second coil; and
perform a wireless communication between the at least one controller and the sensor cartridge via the first sensor and the second sensor using the first light communication tube and the second light communication tube.

2. The sensing device of claim 1, wherein the first coil corresponds to a transmitter coil and the second coil corresponds to a receiver coil.

3. The sensing device of claim 1, wherein the first sensor and second sensor correspond to infrared data association (IrDA) sensors, wherein the first sensor and the second sensor are configured to transmit and receive optical signals through the first light communication tube and the second light communication tube to establish the wireless communication between the at least one controller and the sensor cartridge.

4. The sensing device of claim 3, wherein the at least one controller is configured to transmit the pulse of the predefined frequency to the first coil for a predefined period of time.

5. The sensing device of claim 3, wherein the at least one controller is configured to apply the constant frequency of power to the sensor cartridge via the first coil and the second coil for powering the sensor cartridge.

6. The sensing device of claim 3, wherein a distal end of the first light communication tube is aligned with a proximal end of the second light communication tube to provide a light communication path for transmitting and receiving the optical signal from the first sensor to the second sensor and/or from the second sensor to the first sensor.

7. The sensing device of claim 3, wherein the first light communication tube and the second light communication tube each correspond to a tempered glass tube to provide a flame-resistant path for transmitting and receiving the optical signal.

8. The sensing device of claim 1, wherein the first light communication tube is encased within a flame proof layer, wherein the flame proof layer is made of at least one of an epoxy or cement material;
wherein the first light communication tube and the first flame proof layer are enclosed within a protective shell to prevent the first light communication tube and the first flame proof layer from being displaced; and
wherein the protective shell is at least partially enclosed within a transmitter enclosure via an adhesive to provide flame resistance to the protective shell.

9. A method comprising:
measuring, via at least one controller electrically coupled to a sensor retainer and communicatively coupled to a sensor cartridge, a current flowing through a first coil of the sensor retainer based at least on transmitted pulse of a predefined frequency;
determining, via the at least one controller, if the measured current is above a predefined threshold value;
applying, via the at least one controller, a constant frequency of power to the sensor cartridge via the first coil and a second coil of the sensor cartridge, if the measured current is above the predefined threshold value; and
performing, via the at least one controller, a wireless communication between the at least one controller and the sensor cartridge via a first sensor of the sensor retainer and a second sensor of the sensor cartridge using a first light communication tube of the sensor retainer and a second light communication tube of the sensor cartridge.

10. The method of claim 9, wherein the first coil corresponds to a transmitter coil and the second coil corresponds to a receiver coil.

11. The method of claim 9, wherein the first sensor and the second sensor correspond to Infrared data association (IrDA) sensors; and
wherein the method further comprises transmitting and receiving, via the first sensor and the second sensor, optical signals through the first light communication tube and the second light communication tube to establish the wireless communication between the at least one controller and the sensor cartridge.

12. The method of claim 11, further comprising transmitting, via the at least one controller, the pulse of the predefined frequency to the first coil for a predefined period of time.

13. The method of claim 11, further comprising applying, via the at least one controller, the constant frequency of power to the sensor cartridge via the first coil and the second coil, for powering the sensor cartridge.

14. The method of claim 11, further comprising providing, via a distal end of the first light communication tube aligned with a proximal end of the second light communication tube, a light communication path for transmitting and receiving the optical signal from the first sensor to the second sensor and/or from the second sensor to the first sensor.

15. The method of claim 11, wherein the first light communication tube and the second light communication tube each correspond to a tempered glass tube to provide a flame-resistant path for transmitting and receiving the optical signal.
